# EUROPÄISCHE PATENTSCHRIFT

(11) **EP 2 502 558 B1**
(45) Veröffentlichungstag und Bekanntmachung des Hinweises auf die Patenterteilung: **26.09.2018**
(21) Anmeldenummer: 12159485.7
(22) Anmeldetag: 14.03.2012
(51) Int. Cl.: A61B 5/06, A61B 34/30

(54) **Medizinischer Arbeitsplatz**
Medical workstation
Poste de travail médical

(30) Priorität: 22.03.2011 DE 102011005917
(43) Veröffentlichungstag der Anmeldung: 26.09.2012
(73) Patentinhaber: KUKA Deutschland GmbH, 86165 Augsburg (DE)
(72) Erfinder: Berke, Ralph, 86497 Horgau (DE)
(74) Vertreter: Ege Lee & Partner Patentanwälte PartGmbB

(56) Entgegenhaltungen:
- WO-A2-03/077101
- WO-A2-2005/039391
- WO-A2-2009/045827
- WO-A2-2009/045885
- US-A1- 2002 120 188
- US-A1- 2005 033 161
- US-A1- 2005 107 808
- US-B1- 6 626 832

## Beschreibung

Die Erfindung betrifft einen medizinischen Arbeitsplatz mit einem Roboter.
Die WO 2009/065827 A1 offenbart einen medizinischen Arbeitsplatz mit einem Roboter. Der Roboter umfasst einen Roboterarm und eine Steuervorrichtung zum Bewegen des Roboterarms. An einer Befestigungsvorrichtung des Roboterarms ist z.B. ein Endoskop befestigt, mittels dem ein Lebewesen behandelt werden soll. Um die Positionen und Orientierungen des Lebewesens relativ zum Roboterarm zu ermitteln, ist ein Navigationssystem vorgesehen.
Die WO 2009/045827 A2 offenbart einen medizinischen Arbeitsplatz mit einem Roboterarm und einer bildgebenden Vorrichtung, mittels derer Bilder vom Inneren eines Patienten erstellt werden können. Der medizinische Arbeitsplatz ist für eine Operationsnavigation eingerichtet. Für die Operationsnavigation umfasst der medizinische Arbeitsplatz ein Tool Tracking System, das vorgesehen ist, aufgrund der aufgenommenen Bilder bzw. einer Analyse derselben den Roboterarm zu bewegen, sodass sich z.B. das am Roboterarm befestigte Instrument automatisch zu einem zu behandelnden Tumor bewegt. Die Aufgabe der Erfindung ist es, einen verbesserten medizinischen Arbeitsplatz mit einem Roboter zu schaffen.
Die Aufgabe der Erfindung wird gelöst durch einen medizinischen Arbeitsplatz gemäß dem unabhängigen Anspruch 1. Weitere Ausführungsformen sind in den abhängigen Ansprüchen offenbart.

Der erfindungsgemäße medizinische Arbeitsplatz umfasst demnach das medizinische Instrument und die bildgebende Vorrichtung. Das medizinische Instrument ist vorgesehen, für die Behandlung des Lebewesens zumindest teilweise in dessen Innere eingeführt zu werden. Das medizinische Instrument ist z.B. eine Kanüle, die eingerichtet ist, Fettgewebe des Lebewesens abzusaugen.
Mittels der bildgebenden Vorrichtung können Bilddatensätze vom Inneren des Lebewesens während der Behandlung, also während das medizinische Instrument zumindest teilweise in das Innere des Lebewesens eingeführt ist, aufgenommen werden. Die aufgenommenen Bilddatensätze können z.B. verwendet werden, den Bilddatensätze zugeordnete Bilder mittels einer Anzeigevorrichtung anzuzeigen.
Um nun stets oder zumindest ab und zu aktuelle Bilddatensätze insbesondere von dem Bereich des Inneren des Lebewesens zu erhalten, in dem zumindest teilweise das medizinische Instrument eingeführt ist, weist der erfinderische medizinische Arbeitsplatz den Roboter auf, der eingerichtet ist, den Roboterarm derart zu bewegen, sodass die am Roboterarm befestigte bildgebende Vorrichtung der Bewegung des medizinischen Instruments folgt oder das am Roboterarm befestigte medizinischen Instrument der Bewegung der bildgebenden Vorrichtung folgt. Führt z.B. ein Arzt das medizinsische Instrument manuell zumindest teilweise in das Innere des Lebewesens ein, so folgt die bildgebende Vorrichtung, bewegt durch den Roboterarm insbesondere stets der Bewegung des manuell geführten medizinischen Instruments.

Alternativ kann es vorgesehen sein, dass wenn z.B. der Arzt die bildgebende Vorrichtung manuell führt, der Roboter das medizinsische Instrument automatisch derart bewegt, sodass das medizinische Instrument automatisch der Bewegung der bildgebende Vorrichtung folgt.

Die bildgebende Vorrichtung ist z.B. eingerichtet, Ultraschallbilder zugeordnete Bilddatensätze vom Inneren des Lebewesens zu erstellen. Ein Ultraschallgerät kann relativ klein ausgeführt werden und kann somit gegebenenfalls relativ einfach mittels des Roboters bewegt werden. Außerdem erzeugt ein Ultraschallgerät keine gesundheitsgefährdeten Strahlungen wie z.B. eine Röntgenvorrichtung.

Gemäß einer Ausführungsform des erfindungsgemäßen medizinischen Arbeitsplatzes weist dieser ein mit der Steuervorrichtung des Roboters verbundenes Navigationssystem auf, das eingerichtet ist, die Positionen und /oder Lagen des Roboterarms, des medizinischen Instruments und/oder der bildgebenden Vorrichtung zu ermitteln, aufgrund dessen die Steuervorrichtung den Roboterarm bewegt, sodass die am Roboterarm befestigte bildgebende Vorrichtung der Bewegung des medizinischen Instruments bzw. das am Roboterarm befestigte medizinischen Instrument der Bewegung der bildgebenden Vorrichtung folgt.

Navigationssysteme sind in der Medizintechnik beispielsweise aus der WO 2009/065827 A1 allgemein bekannt. Navigationssysteme umfassen eine Erfassungsvorrichtung, die beispielsweise eine optische Erfassungsvorrichtung, insbesondere eine Kamera, ein Lasertrackingsystem, Projektoren für ein strukturiertes Licht oder Linienprojektoren aufweisen können. Die Erfassungsvorrichtung ist eingerichtet, in allgemein bekannter Weise am Objekt, insbesondere an der Oberfläche des Objekts, angeordnete Marker oder markante Stellen der Oberfläche des Objekts zu erfassen. Aufgrund der Erfassung der Marker bzw. der markanten Stellen kann eine Rechenvorrichtung des Navigationssystems in im Wesentlichen allgemein bekannter Weise die Positionen des Roboterarms, des medizinischen Instruments und/oder der bildgebenden Vorrichtung
und gegebenenfalls dessen Orientierung bestimmen. Somit kann es dem erfinderischen medizinischen Arbeitsplatz ermöglicht werden, z.B. die aktuelle Position und/oder Lage (=Position und Orientierung) z.B. des manuell geführten medizinischen Instrument zu bestimmen, um entsprechend die am Roboterarm befestigte bildgebende Vorrichtung nachzuführen.

Der erfindungsgemäße medizinische Arbeitsplatz kann eine Anzeigevorrichtung aufweisen, die vorgesehen ist, ein dem Bilddatensatz zugeordnetes Bild visuell darzustellen. Somit kann die das Lebewesen behandelnde Person in relativ einfacher Weise das zumindest teilweise in das Innere des Lebewesens eingeführte medizinische Instrument beobachten.

Nach einer Variante des erfindungsgemäßen medizinischen Arbeitsplatzes ist dieser eingerichtet, den Bilddatensatz auf für die Behandlung des Lebewesens mit dem medizinischen Instrument unerwünschte und/oder erwünschte Bereiche zu analysieren.

Dann kann es in vorteilhafter Weise vorgesehen sein, dass der erfindungsgemäße medizinische Arbeitsplatz eingerichtet ist, im mittels der Anzeigevorrichtung dargestellten Bild erwünschte und/oder unerwünschte dargestellte Bereiche zu kennzeichnen. Dadurch kann die das Lebewesen mit dem medizinischen Instrument behandelnde Person besser erkennen, wenn das zumindest teilweise in das Innere eingeführte medizinische Instrument den erwünschten Bereich verlässt oder zumindest droht zu verlassen. Die entsprechenden Bereiche können z.B. farblich gekennzeichnet sein.

Um die das Lebewesen behandelnde Person gegebenenfalls zu warnen, kann nach einer Variante des erfindungsgemäßen medizinischen Arbeitsplatz dieser eingerichtet sein, aufgrund des analysierten Bilddatensatzes ein Warnsignal, insbesondere ein akustisches Warnsignal zu erzeugen, wenn sich das medizinische Instrument außerhalb des erwünschten Bereichs befindet oder droht, diesen zu verlassen.
Um die das Lebewesen behandelnde Person gegebenenfalls zu warnen, kann nach einer Variante des erfindungsgemäßen medizinischen Arbeitsplatzes dessen medizinisches Instrument einen Vibrationsgeber aufweisen und der erfindungsgemäße medizinische Arbeitsplatz eingerichtet sein, aufgrund des analysierten Bilddatensatzes den Vibrationsgeber zu aktivieren, wenn sich das medizinische Instrument außerhalb des erwünschten Bereichs befindet oder droht, diesen zu verlassen. Nach einer weiteren Ausführungsform des erfindungsgemäßen medizinischen Arbeitsplatzes ist dieser eingerichtet, aufgrund des analysierten Bilddatensatzes eine Bewegung des mit dem medizinischen Instrument versehenen Roboterarms zu stoppen oder zu verhindern, wenn sich das medizinische Instrument außerhalb des erwünschten Bereichs befindet oder droht, diesen zu verlassen. Bewegt somit beispielsweise die das Lebewesen behandelnde Person manuell die bildgebende Vorrichtung und führt der Roboter automatisch das medizinische Instrument der Bewegung der bildgebenden Vorrichtung nach, so stoppt gemäß dieser Variante automatisch der Roboter die Bewegung des medizinischen Instruments, wenn dieses droht, einen Bereich zu betreten, den es nicht betreten soll.

Der erfindungsgemäße medizinische Arbeitsplatz weist einen weiteren Roboter mit einem, mehrere, nacheinander angeordnete Glieder aufweisenden Roboterarm und mit einer zum Bewegen des weiteren Roboterarms vorgesehenen weiteren Steuervorrichtung auf, wobei einer der Roboterarme vorgesehen ist, die bildgebende Vorrichtung zu bewegen, und der andere Roboterarme vorgesehen ist, das medizinische Instrument zu bewegen.

Die beiden Steuervorrichtungen sind derart miteinender gekoppelt, sodass bei einer Bewegung einer der Roboterarme der andere Roboterarme automatisch eine Bewegung derart ausführt, sodass das medizinische Instrument einer Bewegung der bildgebenden Vorrichtung oder die bildgebende Vorrichtung einer Bewegung des medizinischen Instruments folgt. Um dies zu erreichen, können z.B. die beiden Steuervorrichtungen als Master-Slave System ausgebildet sein.

Gemäß des erfindungsgemäßen medizinischen Arbeitsplatzes ist einer der Roboterarme manuell bewegbar, und der andere Roboterarm folgt, gesteuert durch seine Steuervorrichtung, automatisch der Bewegung des manuell bewegten Roboterarms. Der fragliche Roboterarm wird durch manuelles Führen. bewegt. Wenn der manuell bewegbare Roboterarm mit dem medizinischen Instrument versehen ist, kann nach einer weiteren Ausführungsform des erfindungsgemäßen medizinischen Arbeitplatzes dieser eingerichtet sein, eine Bewegung des mit dem medizinischen Instruments versehenen Roboterarms zu verhindern oder zumindest zu erschweren, wenn sich das medizinische Instrument außerhalb des erwünschten Bereichs befindet oder droht, diesen zu verlassen. Der erwünschte Bereich bzw. der unerwünschte Bereich kann insbesondere mittels Analysieren des Bilddatensatzes erkannt werden.

Insbesondere wenn der erfindungsgemäße medizinische Arbeitsplatz zum Absaugen von Fettgewebe des Lebewesens verwendet werden soll, kann durch den erfindungsgemäßen medizinischen Arbeitsplatz die Sicherheit und die Qualität des Fettabsaugens erhöht werden. Mit der bildgebenden Vorrichtung wie z.B. dem Ultraschall können insbesondere Fettgewebe relativ gut und effizient dargestellt werden. Dadurch kann die abzusaugende beabsichtigte Fettschicht beispielsweise visualisiert werden, um mit dem als Kanüle ausgebildeten medizinischen Instrument anhand der Bilddaten relativ exakt die relevante Fettschicht absaugen zu können.

Ausführungsbeispiele der Erfindung sind exemplarisch in den beigefügten schematischen Zeichnungen dargestellt. Es zeigen:
- Fig. 1: einen medizinischen Arbeitsplatz mit einem Roboter,
- Fig. 2: ein Ultraschallbild,
- Fig. 3: einen medizinischen Arbeitsplatz mit zwei Robotern und
- Fig. 4: einen weiteren medizinischen Arbeitsplatz mit einem Roboter.

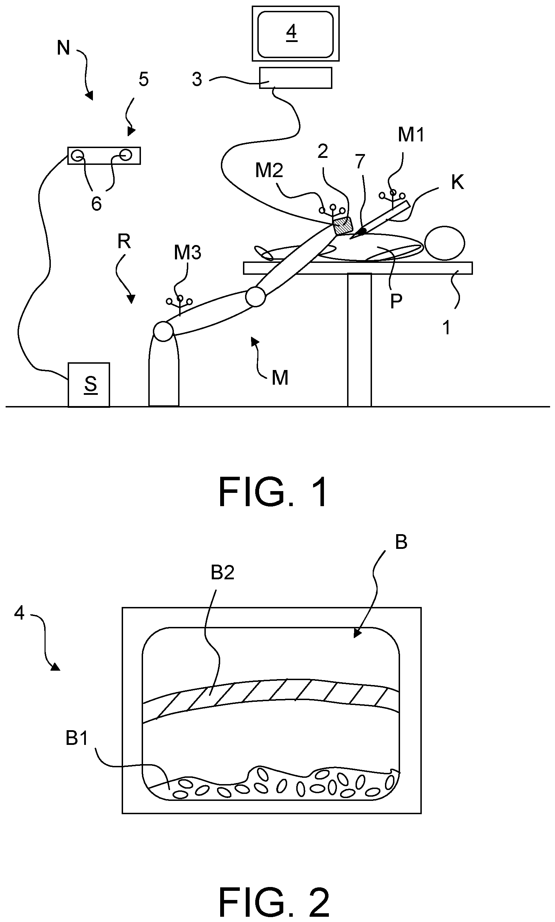

Die Fig. 1 zeigt einen medizinischen Arbeitsplatz mit einer Patientenliege 1, auf der ein zu behandelndes Lebewesen, beispielsweise eine Person P, liegt. Der medizinische Arbeitsplatz weist ferner eine Kanüle K auf, mittels derer eine Fettabsaugung an der Person P durchgeführt werden soll. Die Kanüle K wird manuell von einem nicht näher dargestellten Arzt manuell geführt. Die Kanüle K ist ein Beispiel eines medizinischen Instruments, das in das Lebewesen eingeführt werden kann.

Der medizinische Arbeitsplatz weist ferner einen Roboter R mit einem Roboterarm M und einer Steuervorrichtung S auf. Der Roboterarm M umfasst mehrere, hintereinander folgende Glieder, die mittels Gelenke verbunden und relativ zueinander bezüglich Achsen beweglich sind. An einem Ende des Roboterarms M ist ein Ultraschallwandler 2 befestigt oder in den Roboterarm M integriert. Der Ultraschallwandler 2 ist mit einem Rechner 3 des medizinischen Arbeitsplatzes verbunden und ist eingerichtet, Ultraschallbilder B vom Inneren der Person P bzw. den Ultraschallbildern B zugeordnete Bilddatensätze zu erzeugen. Die Ultraschallbilder B können mittels eines mit dem Rechner 3 verbundenen Monitors 4 dargestellt werden. Eines der Ultraschallbilder B ist in der Fig. 2 dargestellt. Es zeigt z.B. abgebildetes Fettgewebe B1 und die abgebildete Hautoberfläche B2 der Person P.

Der Roboterarm M weist Antriebe, insbesondere elektrische Antriebe auf, welche mit der Steuervorrichtung S verbunden sind. Mittels der Antriebe kann der Roboterarm M bzw. dessen Glieder relativ zueinander und gesteuert durch die Steuervorrichtung S bzw. durch ein auf der Steuervorrichtung S laufendes Rechnerprogramm bewegt werden. Insbesondere ist es dadurch möglich, dass der Ultraschallwandler 2 eine vorgegebene Position und Orientierung im Raum einnimmt. Gegebenfalls werden die Antriebe durch die Steuervorrichtung S geregelt.

Im Falle des vorliegenden Ausführungsbeispieles umfasst der medizinische Arbeitsplatz ein Navigationssystem N. Navigationssysteme als solche sind dem Fachmann u.a. aus der WO 2009/065827 A1 bekannt. Navigationssysteme können beispielsweise magnetische oder optische Navigationssysteme sein oder auf RFID basieren und werden beispielsweise dafür eingesetzt, die Position und gegebenenfalls die Orientierung eines Objekts, beispielsweise des Ultraschallwandlers 2, der Kanüle K oder des Roboterarms M, zu ermitteln.

Im Falle des vorliegenden Ausführungsbeispiels ist die Kanüle mit Markern M1, der Ultraschallwandler 2 mit Markern M2 und der Roboterarm M mit Markern M3 versehen, mittels derer das Navigationssystem N die Positionen bzw. Lagen, d.h. Positionen und Orientierungen, der Kanüle K, des Ultraschallwandlers 2 und des Roboterarms M im Raum bestimmen kann.

Im Falle des vorliegenden Ausführungsbeispieles weist das Navigationssystem N eine Erfassungsvorrichtung 5 auf, die beispielsweise eine Stereokamera 6 umfasst. Die Stereokamera 6 ist eingerichtet, Bilder von den Markern M1, M2, M3 aufzunehmen.

Die Erfassungsvorrichtung 5 ist im Falle des vorliegenden Ausführungsbeispieles mit der Steuervorrichtung S des Roboters R verbunden, auf der ein Rechnerprogramm läuft, das die mittels der Stereokamera 6 aufgenommenen Bilder der Marker M1, M2, M3 in allgemein bekannter Weise auswertet und aufgrund der Auswertung die Position der Marker M1, M2, M3 und somit die Positionen der Kanüle K, des Ultraschallwandlers 2 und des Roboterarms M im Raum bestimmt. Diese Auswertung kann auch von der Erfassungsvorrichtung 5 durchgeführt werden, die dann das Ergebnis der Auswertung an die Steuervorrichtung S übermittelt.

Somit ist es der Steuervorrichtung S bzw. einem auf der Steuervorrichtung S laufenden Rechnerprogramm möglich, die Antriebe des Roboterarms M derart anzusteuern, sodass dieser den Ultraschallwandler 2 derart bewegt bzw. nachführt, sodass der Ultraschallwandler 2 und die Kanüle K einen vorgegebenen Abstand einhalten. Somit wird es ermöglicht, dass während der Behandlung der Person P mittels der Kanüle K der Ultraschallwandler 2 Bilddatensätze erzeugt, die Ultraschallbildern B insbesondere von dem Bereich der Person P vor der Kanüle K zugeordnet sind, sodass diese Ultraschallbilder B mittels des Monitors 4 dem die Kanüle 2 betätigenden Arzt dargestellt werden. Die Ultraschallbilder B stellen demnach online die vor der Kanüle K liegende Gewebeschicht der Person P auf dem Monitor 4 dar. Der Arzt kann dann beispielsweise entscheiden, wie und wohin er die Kanüle K zur Fettabsaugung des Fettgewebes bewegen möchte. Insbesondere kann es vorgesehen sein, dass die Steuervorrichtung S den Roboterarm M bzw. dessen Antriebe stets derart ansteuert, dass der Ultraschallwandler 2 stets der individuellen Bewegung der Kanüle 2 bzw. dessen Spritze folgt. Dem Arzt kann es dadurch ermöglicht sein, dass er auf dem Monitor 4 relativ genau sieht, in welcher Gewebeschicht der Person P sich die Kanüle K gerade befindet.

Auf dem Rechner 3 kann ein Bildverarbeitungsprogramm laufen, das die mittels des Ultraschallwandlers 2 erzeugten Bilddatensätze derart aufbereitet, dass im Ultraschallbild B abgebildetes Fettgewebe B1 z.B. farblich von anderen Bereichen der Person P gekennzeichnet wird. Entsprechend abgebildetes Gewebe kann z.B. mittels Ultraschall-Elastographie erkannt werden. Es ist auch möglich, dass Bereiche der Person P, die nicht mittels der Kanüle K behandelt werden sollen, unterschiedlich im Ultraschallbild B eingefärbt oder markiert dargestellt werden.

So kann es auch vorgesehen sein, dass insbesondere das auf dem Rechner 3 laufende Bildverarbeitungsprogramm erkennt, wenn insbesondere die Spitze der Kanüle K sich einem unerlaubten Bereich, also sich einem Bereich außerhalb des Fettgewebes nähert. Dann ist es möglich, dass der Rechner 3 ein Signal erzeugt, aufgrund dessen der Arzt z.B. akustisch gewarnt wird. Es ist aber auch möglich, dass die Kanüle K einen Vibrationsgeber aufweist, um den Arzt bei manueller Führung vor kritischen Bereichen zu warnen. In diesem Fall ist z.B. der Rechner 3 mit der Kanüle K bzw. dem Vibrationsgeber verbunden.

Es ist auch möglich, dass insbesondere an der Spitze der Kanüle K ein Sensor 7 vorgesehen ist, der insbesondere mit dem Navigationssystem N verbunden ist. Der Sensor 7 kann z.B. eine verbesserte Bestimmung der Position und gegebenenfalls der Orientierung der Kanüle K, insbesondere deren Spitze erlauben. Der Sensor 7 kann z.B. auf RFID basieren.

Der Sensor 7 kann aber auch derart ausgeführt sein, dass er die aktuelle Gewebeart erkennt und z.B. diese Information an den Rechner 3 übermittelt. Die Übermittlung kann z.B. drahtlos z.B. über Funk oder auch kabelgebunden erfolgen. Die vom Sensor 7 stammende Information kann beispielsweise für die Darstellung des Ultraschallbildes B verwendet werden.

Auch könnte der medizinische Arbeitsplatz derart eingerichtet sein, dass z.B. für eine Qualitätssicherung der Eingriff beispielsweise mittels des Rechners 3 dokumentiert wird.

Es ist auch möglich, dass vor dem Eingriff ein präoperativer Bilddatensatz von der Person P aufgenommen wird. Der präoperative Bilddatensatz ist insbesondere ein dreidimensionaler Bilddatensatz und bildet insbesondere den Bereich des Eingriffs ab. Der präoperative Bilddatensatz wird insbesondere mit einem medizintechnischen Gerät, beispielsweise mit einem Magnetresonanzgerät aufgenommen und kann während des Eingriffs mit dem mittels des Ultraschallwandlers 2 aufgenommenen Bilddatensatz verschmolzen oder diesem überlagert sein, um ein modifiziertes Bild zu erzeugen, das anstatt des Ultraschallbildes B am Monitor 4 angezeigt wird.

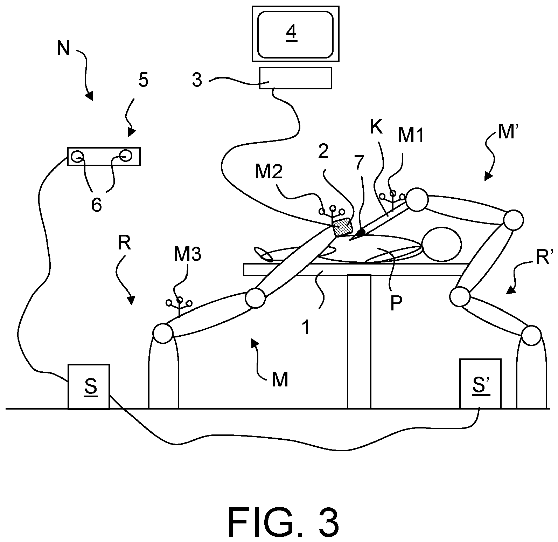

Die Fig. 3 zeigt einen weiteren medizinischen Arbeitsplatz. Wenn nicht anders beschrieben, dann sind Bestandteile des in der Fig. 3 gezeigten medizinischen Arbeitsplatzes, die mit Bestandteilen des medizinischen Arbeitsplatzes der Fig. 1 im Wesentlichen bau- und funktionsgleich sind, mit denselben Bezugszeichen versehen.

Der in der Fig. 3 gezeigte medizinische Arbeitsplatz unterscheidet sich von dem in der Fig. 1 gezeigten medizinischen Arbeitsplatz dadurch, dass dieser einen zweiten Roboter R' umfasst. Der weitere Roboter R' weist einen weiteren Roboterarm M' und eine weitere Steuervorrichtung S' auf. Der weitere Roboterarm M' umfasst mehrere, hintereinander folgende Glieder, die mittels Gelenke verbunden und relativ zueinander bezüglich Achsen beweglich sind. An einem Ende des weiteren Roboterarms M' ist die Kanüle K befestigt. Dazu umfasst der weitere Roboterarm M' eine geeignete Befestigungsvorrichtung z.B. in Form eines Flansches.

Der weitere Roboterarm M' weist Antriebe, insbesondere elektrische Antriebe auf, welche mit der weiteren Steuervorrichtung S' verbunden sind. Mittels seiner Antriebe kann der weitere Roboterarm M' bzw. dessen Glieder relativ zueinander gesteuert durch die weitere Steuervorrichtung S' bzw. durch ein auf der weiteren Steuervorrichtung S' laufendes Rechnerprogramm bewegt werden. Insbesondere ist es dadurch möglich, dass die Kanüle K eine vorgegebene Position und Orientierung im Raum einnimmt. Gegebenfalls werden die Antriebe durch die weitere Steuervorrichtung S' geregelt. Somit wird die Kanüle K vom weiteren Roboter R' geführt.

Somit führt beim medizinischen Arbeitsplatz der Fig. 3 der Roboter R den Ultraschallwandler 2 und der weitere Roboter R' die Kanüle K. Die beiden Roboter R, R' bzw. ihre Steuervorrichtungen S, S' sind im Falle des vorliegenden Ausführungsbeispiels in einem Regelkreis gekoppelt, sodass der die Kanüle K bewegende Roboter R' die Kanüle K nur im erlaubten Fettgewebebereich bewegt. Mittels Bildverarbeitung werden erlaubte und nicht erlaubte Bereiche automatisch im Ultraschallbild B identifiziert und am Monitor 4 angezeigt. Die Bewegung des weiteren Roboters R' kann auch automatisch über virtuelle Wände so begrenzt sein, dass die Führung der Kanüle K nur im erlaubten Fettgewebe-Bereich möglich ist. Es ist auch möglich, dass eine gemeinsame Steuervorrichtung, z.B. die Steuervorrichtung S, beide Roboterarme M, M' ansteuert.

Um die beiden Roboter R, R' miteinander zu koppeln, können deren Steuervorrichtungen S, S' als Master-Slave Systeme ausgeführt sein, sodass beispielsweise für einen automatisierten Eingriff die weitere Steuervorrichtung S' den weiteren Roboterarm R' derart bewegt, sodass die Kanüle K eine vorgegebene Bewegung ausführt. Aufgrund der Kopplung der beiden Steuervorrichtungen S, S' wird es der Steuervorrichtung S ermöglicht, den Roboterarm M und somit den Ultraschallwandler 2 derart zu bewegen, dass dieser den vorgegebenen Abstand zur Kanüle K aufweist. In diesem Fall kann gegebenenfalls der medizinische Arbeitsplatz der Fig. 3 ohne das Navigationssystem N auskommen.

Anstelle des vollautomatisierten medizinischen Arbeitsplatzes der Fig. 3 kann es auch vorgesehen sein, dass zwar zwei Roboter R, R' vorgesehen sind, der Arzt während des Eingriffs jedoch einen der beiden Roboterarme M, M', vorzugsweise den weiteren Roboterarm M', an dem die Kanüle K befestigt ist, manuell bewegt. Insbesondere kann es vorgesehen sein, dass der Arzt den fraglichen Roboterarm M, M' manuell führt, indem er z.B. an der Struktur des entsprechenden Roboterarms M, M' zieht oder drückt. Es ist aber auch möglich, dass er den fraglichen Roboterarm M, M' manuell bewegt, indem er ein nicht näher dargestelltes, dem Fachmann jedoch allgemein bekanntes Bedienhandgerät benützt, das mit der entsprechenden Steuervorrichtung S, S' verbunden ist.

Aufgrund der Kopplung der beiden Steuervorrichtungen S, S' bewegt der Roboter R bzw. sein Roboterarm M den Ultraschallwandler 2 automatisch in dem vorgegeben Abstand zur Spitze der Kanüle K. In dieser Ausführungsform kann es im Falle des vorliegenden Ausführungsbeispiels dann vorgesehen sein, dass der Arzt den weiteren Roboterarm M' nicht mehr oder nur noch mit größerem Kraftaufwand bewegen kann, sobald die Kanüle K in einen unerlaubten Bereich vordringt bzw. den Fettgewebe Bereich verlässt. Dies wird mittels Bildverarbeitung vom Rechner 3 aufgrund des mittels des Ultraschallwandlers 2 aufgenommenen Bilddatensätze erkannt. In diesem Fall kann gegebenenfalls ohne das Navigationssystem N ausgekommen werden. Es ist auch möglich, dass der Roboterarm M, an dem der Ultraschallwandler 2 befestigt ist, manuell bewegt wird.

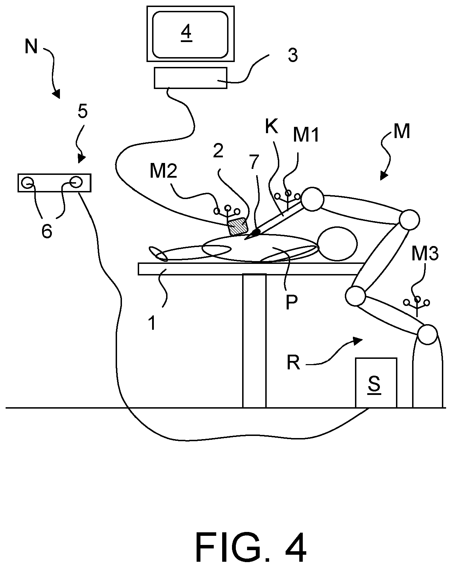

Die Fig. 4 zeigt einen weiteren medizinischen Arbeitsplatz. Wenn nicht anders beschrieben, dann sind Bestandteile des in der Fig. 4 gezeigten medizinischen Arbeitsplatzes, die mit Bestandteilen des medizinischen Arbeitsplatzes der Fig. 1 im Wesentlichen bau- und funktionsgleich sind, mit denselben Bezugszeichen versehen.

Der in der Fig. 4 gezeigte medizinische Arbeitsplatz unterscheidet sich im Wesentlichen vom medizinischen Arbeitsplatz der Fig. 1 dadurch, dass der Roboter R bzw. dessen Roboterarm M nicht den Ultraschallwandler 2, sondern die Kanüle K führt. Der Ultraschallwandler 2 wird dagegen manuell vom Arzt geführt. Aufgrund der von dem Navigationssystem N stammenden Signale wird die Position und gegebenenfalls die Orientierung des Ultraschallwandlers 2 im Raum erkannt, wodurch es der Steuervorrichtung S ermöglicht wird, den Roboterarm M derart zu bewegen, sodass die Kanüle K automatisch der manuellen Bewegung des Ultraschallwandlers 2 folgt, insbesondere stets folgt.

Im Falle des vorliegenden Ausführungsbeispiels ist es vorgesehen, dass wenn die Kanüle K den erlaubten Bereich, also das Fettgewebe der Person P, verlässt, die Bewegung des Roboters R bzw. dessen Roboterarms M durch die Steuervorrichtung S gestoppt wird. Somit kann zumindest weitgehend sichergestellt werden, dass die Kanüle K bzw. dessen Spitze sich ausschließlich innerhalb des Fettgewebes befindet, wodurch die Gefahr eines Absaugens von gesundem Gewebe zumindest verringert wird.

## Patentansprüche

1. Medizinischer Arbeitsplatz, aufweisend ein medizinisches Instrument (K), welches vorgesehen ist, für eine Behandlung eines Lebewesens zumindest teilweise in dessen Innere eingeführt zu werden, eine bildgebende Vorrichtung (2), die eingerichtet ist, während der Behandlung Bilddatensätze vom Inneren des Lebewesens (P) zu erstellen, und einen Roboter (R) mit einem mehrere, hintereinander angeordnete Glieder aufweisenden Roboterarm (M) und mit einer zum Bewegen des Roboterarms (M) vorgesehenen Steuervorrichtung (S), und einen weiteren Roboter (R') mit einem mehrere, nacheinander angeordnete Glieder aufweisenden Roboterarm (M') und mit einer zum Bewegen des weiteren Roboterarms (M') vorgesehenen weiteren Steuervorrichtung (S'), wobei an einem der Roboterarme (M) die bildgebende Vorrichtung (2) und am anderen Roboterarm (M') das medizinische Instrument (2) anordbar ist, einer der Roboterarme (M, M') durch manuelles Führen manuell bewegbar ist und der andere Roboterarm (M'), gesteuert durch seine Steuervorrichtung (S'), automatisch der Bewegung des manuell bewegten Roboterarms (M, M') folgt, sodass die am entsprechenden Roboterarm (M) befestigte bildgebende Vorrichtung (2) einer Bewegung des mittels des manuell bewegten Roboterarms (M') bewegten medizinischen Instruments (2) folgt oder das am entsprechenden Roboterarm (M') befestigte medizinischen Instrument (K) einer Bewegung des mittels des manuell bewegten Roboterarms (M) der bildgebenden Vorrichtung (2) folgt.

2. Medizinischer Arbeitsplatz nach Anspruch 1, bei dem die beiden Steuervorrichtungen (S, S') derart miteinander gekoppelt sind, sodass bei einer Bewegung einer der Roboterarme (M) der andere Roboterarm (M') automatisch eine Bewegung derart ausführt, sodass das medizinische Instrument (K) einer Bewegung der bildgebenden Vorrichtung (2) oder die bildgebende Vorrichtung (2) einer Bewegung des medizinischen Instruments (K) folgt.

3. Medizinscher Arbeitsplatz nach Anspruch 1 oder 2, bei dem die bildgebende Vorrichtung (2) eingerichtet ist, Ultraschallbilder (B) zugeordnete Bilddatensätze vom Inneren des Lebewesens (P) zu erstellen, und/oder das medizinische Instrument als eine Kanüle (K) zum Absaugen von Fettgewebe des Lebewesens (P) ausgebildet ist.

4. Medizinscher Arbeitsplatz nach einem der Ansprüche 1 bis 3, aufweisend ein mit der Steuervorrichtung (S) des Roboters (R) verbundenes Navigationssystem (N), das eingerichtet ist, die Positionen und /oder Lagen des Roboterarms (M), des medizinischen Instruments (K) und/oder der bildgebenden Vorrichtung (2) zu ermitteln, aufgrund dessen die Steuervorrichtung (S) den Roboterarm (M) bewegt, sodass die am Roboterarm (M) befestigte bildgebende Vorrichtung (2) der Bewegung des medizinischen Instruments (2) bzw. das am Roboterarm (M) befestigte medizinische Instrument (K) der Bewegung der bildgebenden Vorrichtung (2) folgt.

5. Medizinscher Arbeitsplatz nach einem der Ansprüche 1 bis 4, der eingerichtet ist, den Bilddatensatz auf für die Behandlung des Lebewesens mit dem medizinischen Instrument (2) unerwünschte und erwünschte Bereiche zu analysieren.

6. Medizinscher Arbeitsplatz nach einem der Ansprüche 1 bis 5, aufweisend eine Anzeigevorrichtung (4), die vorgesehen ist, ein dem Bilddatensatz zugeordnetes Bild (B) visuell darzustellen.

7. Medizinscher Arbeitsplatz nach Anspruch 5 und 6, der eingerichtet ist, im mittels der Anzeigevorrichtung (4) dargestellten Bild (B) die erwünschten und/oder unerwünschten dargestellte Bereiche (B1, B2) zu kennzeichnen.

8. Medizinischer Arbeitsplatz nach einem der Ansprüche 5 bis 7, der eingerichtet ist, aufgrund des analysierten Bilddatensatzes eine Bewegung des mit dem medizinischen Instrument (2) versehenen Roboterarms (M, M') zu stoppen oder zu verhindern, wenn sich das medizinische Instrument (K) außerhalb des erwünschten Bereichs befindet oder droht, diesen zu verlassen.

9. Medizinischer Arbeitsplatz nach einem der Ansprüche 5 bis 8, der eingerichtet ist, aufgrund des analysierten Bilddatensatzes ein Warnsignal, insbesondere ein akustisches Warnsignal zu erzeugen, wenn sich das medizinische Instrument außerhalb des erwünschten Bereichs befindet oder droht, diesen zu verlassen, und/oder dessen medizinisches Instrument (K) einen Vibrationsgeber aufweist und der medizinische Arbeitsplatz eingerichtet ist, aufgrund des analysierten Bilddatensatzes den Vibrationsgeber zu aktivieren, wenn sich das medizinische Instrument außerhalb des erwünschten Bereichs befindet oder droht, diesen zu verlassen.

10. Medizinischer Arbeitsplatz nach einem der Ansprüche 5 bis 9, bei dem der manuell führbare Roboterarm (M') mit dem medizinischen Instrument (K) versehen ist und der medizinische Arbeitsplatz derart eingerichtet ist, eine Bewegung des mit dem medizinischen Instrument (K) versehenen Roboterarms (M') zu verhindern oder zumindest zu erschweren, wenn sich das medizinische Instrument (K) außerhalb des erwünschten Bereichs befindet oder droht, diesen zu verlassen.

## Claims

1. Medical workstation, comprising a medical instrument (K) which is intended to be inserted at least partially inside a living being for treating the latter, an imaging device (2) which is set up to create image data records of the interior of the living being (P) during the treatment, and a robot (R) which has a robot arm (M) having a plurality of members arranged one after the other, and a control device (S) intended for moving the robot arm (M), and having an additional robot (R') with a robot arm (M') having a plurality of members arranged one after the other and with an additional control device (S') intended for moving the additional robot arm (M'), wherein the imaging device (2) can be arranged on one of the robot arms (M) and the medical instrument (2) can be arranged on the other robot arm (M'), one of the robot arms (M, M') can be moved manually by manual guiding and the other robot arm (M'), controlled by its control device (S'), automatically follows the movement of the manually moved robot arm (M, M'), so that the imaging device (2) attached to the robot arm (M) follows a movement of the medical instrument (2) moved by means of the manually moved robot arm (M') or the medical instrument (K) attached to the corresponding robot arm (M') follows a movement of the imaging device (2) manually moved by the robot arm (M)

2. Medical workstation according to claim 1, wherein the two control devices (S, S') are coupled together in such a way that during a movement of one of the robot arms (M) the other robot arm (M') automatically executes a movement in such a way that the medical instrument (K) follows a movement of the imaging device (2) or the imaging device (2) follows a movement of the medical instrument (K).

3. Medical workstation according to claim 1 or 2, wherein the imaging device (2) is set up to create image data records of the interior of the living being (P) assigned to ultrasound images (B) and/or the medical instrument is designed as a cannula (K) for suctioning off fat tissue of the living being (P).

4. Medical workstation according to any of claims 1 to 3, having a navigation system (N) connected to the control device (S) of the robot (R) which is set up to ascertain the positions and/or locations of the robot arm (M), the medical instrument (K) and/or the imaging device (2), on the basis of which the control device (S) moves the robot arm (M), so that the imaging device (2) attached to the robot arm (M) follows the movement of the medical instrument (K) or the medical instrument (K) attached to the robot arm follows the movement of the imaging device (2).

5. Medical workstation according to any of claims 1 to 4, which is set up to analyse the image data record for undesired and desired areas for the treatment of the living being with the medical instrument (2).

6. Medical workstation according to any of claims 1 to 5, having a display device (4) that is intended to visually depict an image (B) assigned to the image data record.

7. Medical workstation according to claim 5 and 6, which is set up to identify the desired and/or undesired depicted areas (B1, B2) in the image (B) depicted by means of the display device (4).

8. Medical workstation according to any of claims 5 to 7, which is set up to stop or prevent a movement of the robot arm (M, M') provided with the medical instrument (K) on the basis of the analysed image data record, if the medical instrument (K) is located outside of the desired area or threatens to leave the latter.

9. Medical workstation according to any of claims 5 to 8, which is set up to produce a warning signal, in particular an acoustic warning signal, on the basis of the analysed image data record, if the medical instrument is located outside of the desired area or threatens to leave the latter, and/or its medical instrument (K) has a vibration emitter, and the medical workstation is set up to activate the vibration emitter on the basis of the analyzed image record if the medical instrument is located outside of the desired area or threatens to leave the latter.

10. Medical workstation according to any of claims 5 to 9, wherein the manually guided robot arm (M') is provided with the medical instrument (K) and the medical workstation is set up to prevent or at least hinder a movement of the robot arm (M') provided with the medical instrument (K) if the medical instrument (K) is located outside of the desired area or threatens to leave the latter.

## Revendications

1. Poste de travail médical, présentant un instrument médical (K) qui est prévu pour être introduit au moins en partie à l'intérieur d'un être vivant pour un traitement de celui-ci, un dispositif d'imagerie (2) qui est conçu pour établir pendant le traitement des jeux de données d'image de l'intérieur de l'être vivant (P), et un robot (R) avec un bras de robot (M) présentant plusieurs membres agencés les uns derrière les autres, et avec un dispositif de commande (S) prévu pour déplacer le bras de robot (M), et un autre robot (R') avec un bras de robot (M') présentant plusieurs membres agencés les uns derrière les autres, et avec un dispositif de commande (S') prévu pour déplacer l'autre bras de robot (M'), le dispositif d'imagerie (2) pouvant être agencé sur un des bras de robot (M) et l'instrument médical (K) pouvant être agencé sur l'autre bras de robot (M), un des bras de robot (M, M') pouvant être déplacé à la main par un guidage manuel et l'autre bras de robot (M'), commandé par son dispositif de commande (S'), suivant automatiquement le mouvement du bras de robot (M, M') déplacé à la main, de telle sorte que le dispositif d'imagerie (2) fixé sur le bras de robot (M) correspondant suit un mouvement de l'instrument médical (K) déplacé au moyen du bras de robot (M') déplacé à la main ou l'instrument médical (K) fixé sur le bras de robot (M') correspondant suit un mouvement du dispositif d'imagerie (2) déplacé au moyen du bras de robot (M) déplacé à la main.

2. Poste de travail médical selon la revendication 1, dans lequel les deux dispositifs de commande (S, S') sont couplés de telle sorte l'un à l'autre que lors d'un mouvement de l'un des bras de robot (M), l'autre bras de robot (M') effectue automatiquement un mouvement de telle sorte que l'instrument médical (K) suit un mouvement du dispositif d'imagerie (2) ou le dispositif d'imagerie (2) suit un mouvement de l'instrument médical (K).

3. Poste de travail médical selon la revendication 1 ou 2, dans lequel le dispositif d'imagerie (2) est conçu pour établir des jeux de données d'image associés à des échographies (B), de l'intérieur de l'être vivant, et/ou dans lequel l'instrument médical est réalisé sous forme d'une canule (K) pour aspirer du tissu graisseux de l'être vivant (P).

4. Poste de travail médical selon l'une des revendications 1 à 3, présentant un système de navigation (N) relié au dispositif de commande (S) du robot (R), qui est conçu pour déterminer les positions et/ou les situations du bras de robot (M), de l'instrument médical (K) et/ou du dispositif d'imagerie (2), sur la base duquel le dispositif de commande (S) déplace le bras de robot (M), de telle sorte que le dispositif d'imagerie (2) fixé sur le bras de robot (M) suit le mouvement de l'instrument médical (K) ou l'instrument médical (K) fixé sur le bras de robot (M) suit le mouvement du dispositif d'imagerie (2), respectivement.

5. Poste de travail médical selon l'une des revendications 1 à 4, qui est conçu pour analyser le jeu de données d'image sur des zones non souhaitées et souhaitées pour le traitement de l'être vivant avec l'instrument médical (K).

6. Poste de travail médical selon l'une des revendications 1 à 5, présentant un dispositif d'affichage (4) qui est prévu pour représenter visuellement une image (B) associée au jeu de données d'image.

7. Poste de travail médical selon les revendications 5 et 6, qui est conçu pour marquer les zones (B1, B2) souhaitées et/ou non souhaitées dans l'image (B) représentée au moyen du dispositif d'affichage (4).

8. Poste de travail médical selon l'une des revendications 5 à 7, qui est conçu pour arrêter ou empêcher un mouvement du bras de robot (M, M') pourvu de l'instrument médical (K), sur la base du jeu de données d'image, lorsque l'instrument médical (K) se trouve en dehors de la zone souhaitée ou menace de quitter celle-ci.

9. Poste de travail médical selon l'une des revendications 5 à 8, qui est conçu pour engendrer un signal d'alarme, en particulier un signal d'alarme acoustique, sur la base du jeu de données d'image, lorsque l'instrument médical se trouve en dehors de la zone souhaitée ou menace de quitter celle-ci, et/ou lorsque son instrument médical (K) présente un émetteur de vibrations et le poste de travail médical est conçu pour activer l'émetteur de vibrations, sur la base du jeu de données d'image, lorsque l'instrument médical se trouve en dehors de la zone souhaitée ou menace de quitter celle-ci.

10. Poste de travail médical selon l'une des revendications 5 à 9, dans lequel le bras de robot (M') susceptible d'être déplacé à la main, est pourvu de l'instrument médical (K) et le poste de travail médical est conçu pour empêcher ou au moins rendre difficile un mouvement du bras de robot (M') pourvu de l'instrument médical (K), lorsque l'instrument médical (K) se trouve en dehors de la zone souhaitée ou menace de quitter celle-ci.
